(19) Europäisches Patentamt
European Patent Office
Office européen des brevets

(11) **EP 2 930 247 A1**

(12) **EUROPEAN PATENT APPLICATION**

(43) Date of publication:
**14.10.2015 Bulletin 2015/42**

(51) Int Cl.:
***C12Q 1/68*** *(2006.01)*       ***G01N 33/576*** *(2006.01)*
***G01N 33/68*** *(2006.01)*

(21) Application number: **14382134.6**

(22) Date of filing: **07.04.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fundació Clinic per a la Recerca Biomèdica (FCRB)**
**08036 Barcelona (ES)**

(72) Inventor: **Ginès, Pere**
**08036 Barcelona (ES)**

(74) Representative: **ABG Patentes, S.L.**
**Avenida de Burgos, 16D**
**Edificio Euromor**
**28036 Madrid (ES)**

(54) **Biomarker of prognosis and acute-on chronic liver failure in cirrhosis**

(57)    The invention relates to methods for determining the risk of a patient suffering from cirrhosis of developing acute on chronic liver failure based on the expression level of Neutrophil Gelatinase-Associated Lipocalin (NGAL) = LCN2 = Lipocalin-2, methods for determining the prognosis of ACLF or cirrhosis or for determining the stage or severity of ACLF or cirrhosis in a subject suffering from said pathology, methods for monitoring the effect of a therapy in a patient suffering from ACLF, method for the differential diagnoses of acute-on chronic-liver failure (ACLF) from acute decompensation of cirrhosis as well as to methods for selecting a patient suffering from ACLF for a therapy aimed at treating the effects of liver failure and/or for selecting a patient for liver transplantation.

**Description**

**FIELD OF THE INVENTION**

The invention relates to the field of liver diseases and, more in particular, to methods for determining the risk of a patient suffering from cirrhosis of developing acute on chronic liver failure (ACLF) based on the expression level of Neutrophil Gelatinase-Associated Lipocalin (NGAL). The invention also relates to methods for determining the prognosis of ACLF or cirrhosis or for determining the stage or severity of ACLF or cirrhosis in a subject suffering from said pathology, methods for monitoring the effect of a therapy in a patient suffering from ACLF, method for the differential diagnoses of acute-on chronic-liver failure (ACLF) from acute decompensation of cirrhosis as well as to methods for selecting a patient suffering from ACLF for a therapy aimed at treating the effects of liver failure and/or for selecting a patient for liver transplantation.

**BACKGOUND OF THE INVENTION**

The appearance of liver failure in a patient with cirrhosis represents a decisive time point both in terms of medical management and prognosis since this condition is frequently associated with rapidly evolving multi-organ dysfunction. The lack of liver detoxification, metabolic and regulatory functions and altered immune response lead to life-threatening complications, such as renal failure, increased susceptibility to infection, hepatic coma and systemic hemodynamic dysfunction. Among the liver diseases, cirrhosis is by far the most frequent reason for hospital admission or liver transplantation. It can be expected that this situation is unlikely to change in the next decade since recent WHO projections have demonstrated that cirrhosis will become the ninth most common cause of death in the Western World by 2015 (World Health Organization. Projections of mortality and burden of disease to 2030). Moreover, only 20% of patients with advanced cirrhosis globally can be treated with liver transplantation owing to the great imbalance between donation and potential recipients.

Cirrhosis progresses over several years. It is first compensated when the disease has not functional symptoms or complications. The occurrence of complications indicated the transition to the so-called decompensated wherein complications such ascites, gastrointestinal bleeding, renal failure, bacterial infections or hepatic encephalopathy appears.

Acute-on-chronic liver failure (ACLF) is an increasingly recognized entity encompassing an acute deterioration of liver function in patients with cirrhosis, either secondary to superimposed liver injury or due to extrahepatic precipitating factors such as infection culminating in the end-organ dysfunction. Occasionally, no specific precipitating event can be found. Although the exact pathophysiology of the development of ACLF remains to be elucidated, unregulated inflammation is thought to be a major contributing factor. A characteristic feature of ACLF is its rapid progression, the requirement for multiple organ supports and high short and medium-term mortality, of 50-90%. Unfortunately, available therapeutic options for ACLF are limited. Liver transplantation remains the only definitive therapy for patients with ACLF, however, as has been mentioned above, limited availability of the donor organs limits its usefulness in the management of patients with ACLF.

Although in theory the currently used working definition of ACLF seems straightforward to use, medical practice is hampered by an often complex clinical picture, the absence of a clear differentiation from a patient with end-stage liver disease and problems in determining the ideal moment and/or necessity or usefulness of certain therapeutic interventions (Katoonizadeh et al., 2010, Gut, 59:1561-1569). With regard to prognosis, ACLF also poses problems since it coalesces both an acute potentially life- threatening insult and a severe chronic underlying disease.

The few available prognostic models for decision making-ACLF for conventional management or liver transplantation are usually far for being satisfactory. Nowadays, the Model for End Stage Liver Disease (MELD) is the scoring system for assessing the severity of chronic liver diseases useful in determining prognosis and prioritizing for receipt of a liver transplant.

However, MELD score has several limitations including interlaboratory variations for measurement of creatinine and international normalized ratio of prothrombin time and systematic adverse female gender bias. Adjustments to the original MELD equation and new scoring systems have been proposed to overcome these limitations, such incorporation of serum sodium measurement (Cholongitas et al., 2010, Nat. Rev. Gastr. And Hepat., 7, 659-668).

Thus, well designated predictive models are necessary to establish an optimal assessment and management for ACLF as well as for selecting patients for liver transplantation.

**SUMMARY OF THE INVENTION**

In a first aspect, the invention relates to an *in vitro* method for determining the risk of developing acute on chronic liver failure (ACLF) in a subject suffering from cirrhosis which comprises:

a) determining the expression level of Neutrophil Gelatinase-Associated Lipocalin (NGAL) in a sample from said subject; and

b) comparing the expression level of NGAL obtained in a) with a reference value

wherein if the expression level of NGAL is increased with respect to the reference value said subject has high risk of developing ACLF.

In another aspect, the invention relates to an *in vitro* method for determining the prognosis of ACLF or cirrhosis or for determining the stage or severity of ACLF or cirrhosis in a subject suffering from said pathology which comprises:

a) determining the expression level of NGAL in a sample from said subject; and

b) comparing the expression level of NGAL obtained in a) with a reference value

wherein if the expression level of NGAL is increased with respect to the reference value, said subject shows bad prognosis of ACLF or cirrhosis or said subject is suffering from advance stage of ACLF.

In another aspect, the invention relates to an *in vitro* method for monitoring the effect of a therapy in a patient suffering from ACLF and being treated with said therapy which comprises:

a) determining the expression level of NGAL in a sample from said subject; and

b) comparing the expression level of NGAL obtained in a) with a the expression level of NGAL in a sample from said subject at earlier point of time,

wherein if the expression level of NGAL is decreased with respect to the expression level of NGAL determined in a sample from said subject at earlier point of time the therapy is being effective.

In another aspect, the invention relates to an *in vitro* method for the differential diagnoses of acute-on chronic-liver failure (ACLF) from acute decompensation of cirrhosis in a subject which comprises:

a) determining the expression level of NGAL in a sample from said subject; and

b) comparing the expression level of NGAL obtained in a) with a reference value

wherein if the expression level of NGAL is increased with respect to the reference value, said subject suffers ACLF.

In another aspect, the invention relates to an *in vitro* method for selecting a patient suffering from ACLF for a therapy aimed at treating the effects of liver failure and/or for selecting a patient for liver transplantation which comprises:

a) determining the expression level of NGAL in a sample from said patient; and

b) comparing the expression level of NGAL obtained in a) with a reference sample

wherein if the expression level of NGAL is increased with respect to the reference sample said patient is a candidate for receiving a therapy aimed at treating the effects of liver failure and/or for liver transplantation.

In another aspect, the invention relates to the use of NGAL for determining the risk of developing ACLF, for determining the prognosis of ACLF or cirrhosis, for determining the stage or severity of ACLF or cirrhosis in a subject, for monitoring the effect of a therapy in a patient suffering from ACLF, for the differential diagnosis of ACLF from acute decompensation of cirrhosis or for selecting a patient suffering from ACLF for a therapy aimed at treating the effects of liver failure and/or for liver transplantation.

**BRIEF DESCRIPTION OF THE DRAWINGS**

**Figure 1.** Plot of individual values of uNGAL vs serum creatinine levels in all the 716 patients included in the study (77 points are outside of the axis limits). The vertical line represents the median value of uNGAL in the overall series (33μg/g creatinine) and the horizontal line represents a serum creatinine level of 1.5 mg/dL.

**Figure 2.** Relationship between 28-day mortality and MELD score in all patients categorized in four groups according to uNGAL values in quartiles.

**DETAILED DESCRIPTION OF THE INVENTION**

The authors of the present invention have surprisingly found that Neutrophil Gelatinase-associated lipocalin (NGAL) is a biomarker having a predictive value of ACLF development in a subject suffering from cirrhosis. Moreover, the authors of the present invention have observed that the determination of the expression level of NGAL improves the accuracy of MELD score in the prediction of the survival of the patient. The authors of the invention have also found that NGAL is a biomarker which discriminates patients suffering from ACLF from patients having acute decompensation of cirrhosis.

First method of the invention

The authors of the invention have found that NGAL is a biomarker which predicts with high accuracy the development of ACLF in patients suffering from cirrhosis.

Thus, in a first aspect, the invention relates to an *in vitro* method for determining the risk of developing acute-on-chronic liver failure (ACLF) in a subject suffering from cirrhosis (hereinafter referred as the "first method of the invention") which comprises:

a) determining the expression level of Neutrophil Gelatinase-Associated Lipocalin (NGAL) in a sample from said subject; and
b) comparing the expression level of NGAL obtained in a) with a reference value

wherein if the expression level of NGAL is increased with respect to the reference value said subject has high risk of developing ACLF.

The expression "determining the risk" or "prediction of the risk", or similar, as used herein, is synonymous of the expression "assessing the risk" or "assessment of the risk", means that the present invention makes it possible to predict, estimate or evaluate the risk of a patient with cirrhosis to developing ACLF. The prediction of risk generally implies that the risk is either increased or reduced. As it will be understood by those skilled in the art, the prediction (or the risk), although preferred to be, need not be correct for 100% of the cirrhosis patients to be evaluated. The term, however, requires that a statistically significant portion of cirrhosis patients can be identified as having an increased probability of having ACLF. Whether a subject is statistically significant can be determined without further ado by the person skilled in the art by using various well known statistic evaluation tools, e.g., determination of confidence intervals, p-value determination, Student's t-test, Mann-Whitney test, etc. Details can be found in Dowdy and Wearden, Statistics for Research, John Wiley & Sons, New York 1983. Preferred confidence intervals are at least 50%, at least 60%, at least 70%, at least 80%, at least 90% at least 95%. The p-values are, preferably 0.05, 0.025, 0.001, 0.0001 or lower.

The term "acute-on-chronic liver failure" or "ACLF" as used herein, refers to a syndrome characterized by acute deterioration of liver function in patients with compensated or decompensated cirrhosis who have failure of one or several organs, as explained below, and a poor short-term survival. According to the CANONIC study, said syndrome is classified in three grade of severity depending on the organ failure and mortality data:

- ACLF grade 1: this grade includes 3 subgroups of patients: (1) patients with single kidney failure, (2) patients with single failure of the liver, coagulation, circulation or respiration who have a serum creatinine level ranging from 1.5 to 1.9 mg/dL and/or mild moderate hepatic encephalopathy, and (3) patients with single cerebral failure who had a serum creatinine level ranging from 1.5 and 1.9 mg/dL. The 28-day and 90-day mortality rates are about 22.1% and 40.7%, respectively.
- ACLF grade 2: this group includes patients with 2 organ failures. The 28-day and 90-day mortality rates are about 32% and 52.3%, respectively.
- ACLF grade 3: this group includes patients with 3 organ failure or more. The 28-day and 90-day mortality rates are about 76.7% and 79.1 %, respectively.

The term "cirrhosis" as used herein, refers to a condition characterized by replacement of liver tissue by fibrosis and regenerative nodules which lead to loss of liver function. Ascites (fluid retention in the abdominal cavity) is the most common complication of cirrhosis. It is associated with a poor quality of life, increased risk of infection and poor long-term outcome. Other potentially life-threatening complications are hepatic encephalopathy (confusion and coma) and bleeding from esophageal varices. Cirrhosis has many possible manifestations. These signs and symptoms may be either as a direct result of the failure of liver cells or secondary to the resultant portal hypertension. Effects of portal hypertension include splenomegaly, gastroesophageal varices, and portocollateral circulation as a result of formation of venous collateral veins between portal system and the periumbilical veins as a result of portal hypertension.

Cirrhosis is divided in two clinical categories: compensated and decompensated cirrhosis.

The term "compensated cirrhosis" as used herein, means that the liver is heavily scarred but can still perform many important bodily functions. Patients suffering from compensated cirrhosis experience few or no symptoms and can live without serious clinical complications. Patients at early stages of compensated cirrhosis are characterized by low levels of portal hypertension and lack of esophageal varices. Patients at advanced stages of compensated cirrhosis are characterized by higher levels of portal hypertension and presence of esophageal varices but without ascites and without bleeding.

The term "decompensated cirrhosis" or "acute decompensated cirrhosis" as used herein, means that the liver is extensively scarred and unable to function properly. Patients suffering from decompensated cirrhosis develop a variety of symptoms such as fatigue, loss of appetite, jaundice, weight loss, ascites and/or edema, hepatic encephalopathy and/or bleeding. Patients at early stages of decompensated cirrhosis are characterized by the presence of ascites with or without esophageal varices in a patient that has never bled. Patients at advanced stages of compensated cirrhosis are characterized by more sever ascites alone or in association with bleeding, bacterial infections and/or hepatic encephalopathy.

Complications associated with decompensated cirrhosis such ascites, edema, bleeding problems, bone mass and bone density loss, hepatomegaly, menstrual irregularities in women and gynecomastia in men, impaired mental status, itching, kidney function failure, muscle wasting etc. can be developed.

In a preferred embodiment, first method of the invention relates to determining the risk of developing ACLF in a subject suffering from cirrhosis based on the determination of NGAL levels, wherein the cirrhosis is decompensated.

The term "Neutrophil Gelatinase-Associated Lipocalin", "NGAL" or "lipocailin-2" as used herein, refers to a protein that in humans is encoded by the LCN2 gene. NGAL is involved in innate immunity by sequestrating iron that in turn limits bacterial growth. It is expressed in neutrophils and in low levels in the kidney, prostate and epithelia of the respiratory and alimentary tracts. The complete protein sequence of human NGAL has the Uniprot accession number P80188 (released on 19 February, 2014). Under normal conditions, NGAL levels are low in urine and plasma but in case of acute kidney injury, NGAL is secreted into the blood and urine. In preferred embodiment of the present invention, the term NGAL refers to urine NGAL or uNGAL.

The term "subject" or "patient" as used herein, refers to any animal classified as a mammal and includes but is not restricted to domestic and farm animals, primates and humans, for example, human beings, non-human primates, cows, horses, pigs, sheep, goats, dogs, cats, or rodents. The subject is preferably a male or female human being of any age or race.

The term "sample", as used herein refers to biological material isolated from a subject and therefore includes biological samples. Said sample can contain any biological material suitable for detecting the desired marker and can comprise cells and/or non-cellular material from the subject. In general, a sample can be isolated from any suitable biological tissue or fluid; nevertheless, said sample is preferably a biofluid from the subject under study for putting the present invention into practice. Said biofluid sample can be a urine sample, for example, a micturition urine sample, a sample of bladder wash, a sample of blood, a sample of serum etc., and can be obtained by means of any conventional method. In a particular embodiment, said biofluid is selected from urine or serum. In a preferred embodiment of the present invention, said biofluid is urine.

In a first step, the first method of the invention comprises determining the expression level of NGAL in a sample from the subject under study. As it is used herein, the term "expression level" refers to the value of a parameter that measures the degree of expression of a specific gene. In a particular embodiment, said value can be determined by measuring the mRNA level of the gene of interest or a fragment thereof or by measuring the amount of protein encoded by said gene of interest or a variant thereof.

Virtually any conventional method for detecting and quantifying the expression level of a gene can be used within the framework of the present invention for detecting and quantifying the expression level of a specific gene. By way of non-limiting illustration, the expression level of a gene can be determined by means of quantifying the mRNA level of said gene or by means of quantifying the level of protein encoded by said gene. Methods for determining the amount of mRNA are well-known in the state of the art. For example, the nucleic acid contained in the sample, such as the biofluid sample from the subject under study, is extracted according to conventional methods, for example, by means of using lytic enzymes, chemical solutions or fixing resins. The extracted mRNA can be detected by hybridization (for example by means of Northern blot analysis or DNA or RNA arrays (microarrays) after converting mRNA into labeled cDNA) and/or amplification by means of a enzymatic chain reaction. In general, quantitative or semi-quantitative enzymatic amplification methods are preferred. The polymerase chain reaction (PCR) or quantitative real-time RT-PCR or semi-quantitative RT-PCR technique is particularly advantageous. Primer pairs are preferably designed for the purpose of superimposing an intron to distinguish cDNA amplification from the contamination from genomic DNA (gDNA). Additional primers or probes, which are preferably labeled, for example with fluorescence, which hybridize specifically in regions located between two exons, are optionally designed for the purpose of distinguishing cDNA amplification from the contamination from gDNA. If desired, said primers can be designed such that approximately the nucleotides comprised from the 5' end to half the total length of the primer hybridize with one of the exons of interest, and approximately the nucleotides comprised from the 3' end to half the total length of said primer hybridize with the other exon of interest. Suitable primers can be readily designed by a person skilled in the art. Other amplification methods include ligase chain reaction (LCR), transcription-mediated amplification (TMA), strand displacement amplification (SDA) and nucleic acid sequence based amplification (NASBA). The amount of mRNA is preferably measured quantitatively or semi-quantitatively. Relevant information about conventional methods for quantifying the expression level of a gene can be found, for example, in Sambrook *et al.,* 2001 [Sambrook, J., et al., "Molecular cloning: a Laboratory Manual", 3rd ed., Cold Spring Harbor Laboratory Press, N.Y., Vol. 1-3].

To normalize the expression values of one gene among different samples, comparing the mRNA level of the gene of interest in the samples from the subject object of study with a control RNA level is possible. As it is used herein, a "control RNA" is RNA of a gene the expression level of which does not differ depending on if the subject suffers from ACLF or not; a control RNA is preferably an mRNA derived from a housekeeping gene encoding a protein that is constitutively expressed and carrying out essential cell functions. Illustrative, non-limiting examples of housekeeping genes for use in the present invention include GUSB (beta-glucuronidase), PPIA (peptidyl-prolyl isomerase A), β-2-microglobulin, GAPDH, PSMB4 (proteasome subunit beta type-4), ubiquitin, transferrin receptor, 18-S ribosomal RNA, cyclophilin, tubulin, β-actin, 3-monooxygenase/tryptophan 5-monooxygenase tyrosine activation protein (YWHAZ), etc. If the expression level of NGAL is determined by measuring the expression level of transcription product (mRNA) of said gene in a sample from the subject under study, the sample can be treated to physically or mechanically break up the structure of the tissue or cell for the purpose of releasing the intracellular components into an aqueous or organic solution to prepare the nucleic acids for additional analysis. Care is preferably taken to prevent RNA degradation during the extraction process.

In a particular and preferred embodiment of the invention, the expression level of NGAL is determined by means of determining the expression level of the protein encoded by the NGAL gene or a variant thereof, because increased expression of a gene is usually accompanied by an increase in the amount of corresponding protein, is also possible. The term "variant" as used herein, relates to those variant of human NGAL which appear naturally in other species, i.e. the orthologues of NGAL. Said variants include, without limitation, mouse NGAL, which corresponds to the sequence with accession number NP_032517 dated 12 March 2014 in the NCBI database; pig NGAL, which corresponds to the sequence with accession number NP_001231339 dated 10 January 2014 in the NCBI database; macaque NGAL, which corresponds to the sequence with accession number AFI35541 dated 30 April 2012 in the NCBI database; rat NGAL, which corresponds to the sequence with accession number NP_570097 dated 26 February 2014 in the NCBI database. The natural variants of NGAL suitable for their use in the present invention also derive from said sequence by insertion, substitution or deletion of one or more amino acids and include natural alleles, variants resulting from alternative processing and truncate forms which appear naturally. The term "variant" also includes fragments, isoforms and analogues or derivatives of NGAL. Preferably, variants of NGAL are (i) polypeptides in which one or more amino acid residues are substituted by a preserved or non-preserved amino acid residue (preferably a preserved amino acid residue) and such substituted amino acid may be coded or not by the genetic code, (ii) polypeptides in which there is one or more modified amino acid residues, for example, residues modified by substituent bonding, (iii) polypeptides resulting from alternative processing of a similar mRNA, (iv) polypeptide fragments and/or (iv) polypeptides resulting from NGAL fusion or the polypeptide defined in (i) to (iii) with another polypeptide, such as a secretory leader sequence or a sequence being used for purification (for example, His tag) or for detection (for example, Sv5 epitope tag). The fragments include polypeptides generated through proteolytic cut (including multisite proteolysis) of an original sequence. The variants may be post-translationally or chemically modified. Such variants are supposed to be apparent to those skilled in the art.

Variants according to the present invention includes amino acid sequences that are at least 60%, 65%, 70%, 72%, 74%, 76%, 78%, 80%, 90%, or 95% similar or identical to the original amino acid sequence. The degree of identity between two proteins is determined using computer algorithms and methods that are widely known for the persons skilled in the art. The identity between two amino acid sequences is preferably determined by using the BLASTP algorithm [BLASTManual, Altschul, S., et al., NCBI NLM NIH Bethesda, Md. 20894, Altschul, S., et al., J. Mol. Biol. 215: 403-410 (1990)].

The proteins can be post-translationally modified. For example, post-translational modifications that fall within the scope of the present invention include signal peptide cleavage, glycosylation, acetylation, isoprenylation, proteolysis myristoylation, protein folding and proteolytic processing, etc. Additionally, the proteins may include unnatural amino acids formed by post-translational modification or by introducing unnatural amino acids during translation.

The determination of the amount of a protein corresponding to the expression of a specific gene can be performed using any conventional method for protein detection and quantification, for example by means of an immunoassay, etc. By way of non-limiting illustration, said determination can be performed using antibodies with the capability to bind specifically to the protein to be determined (or fragments thereof with the antigenic determinants) and subsequent quantification of the antigen-antibody complex derivatives. The antibodies can be, for example, polyclonal sera, hybridoma supernatants or monoclonal antibodies, fragments of antibodies, Fv, Fab, Fab' and F(ab')$_2$, scFv, diabodies, triabodies, tetrabodies, humanized antibodies, etc. Said antibodies may (or may not) be labeled with a marker. Illustrative, non-limiting examples of markers that can be used in the present invention include radioactive isotopes, enzymes, fluorophores, chemiluminescent reagents, enzyme cofactors, enzyme substrates, enzyme inhibitors, etc. There is a wide range of well-known assays that can be used in the present invention, such as, for example, assays based on Western-blot or immunoblot techniques, ELISA (enzyme-linked immunosorbent assay), RIA (radioimmunoassay), EIA (enzyme immunoassay), DAS-ELISA (double antibody sandwich ELISA), immunocytochemical or immunohistochemical techniques, etc. Other ways of detecting and quantifying the protein include affinity chromatogaphy, ligand binding assay techniques, particle-enhanced turbidimetric immunoassay (PETIA) etc.

In a particular embodiment, the determination of the expression level of the NGAL protein is carried out by Western blot, ELISA or protein array. In a preferred embodiment, the expression level of NGAL is determined by using a commercial ELISA kit (Bioporto, Gentofe, Denmark.

Briefly, the enzyme-linked immunoabsorbent assay (ELISA) is a technique that uses a solid enzyme immunoassay to detect the presence of a substance, usually an antigen, in a liquid sample or a wet sample. Antigens from the sample are attached to a surface. Then, a further specific antibody is applied over the surface, so it can bind to the antigen. This antibody is linked to an enzyme, and, in the final step, a substance, containing the enzyme's substrate is added. The subsequent reaction produces a detectable signal, most commonly a color change in the substrate.

To normalize the expression values of one protein, i.e. NGAL, among different samples, comparing the protein level of the protein of interest in the samples from the subject object of study with a control protein level is possible. In one particular embodiment, the expression level of NGAL is determined in absolute terms, i.e. by providing the concentration of NGAL in a sample. In another particular embodiment, the expression level of NGAL is measured relative to creatinine concentration. In a particular preferred embodiment, the expression level of NGAL is measured relative to urine creatinine concentration. Said measurement eliminates the problem of urine dilution and gives more accurate reflection of the true protein excretion rate. The term "creatinine" as used herein, refers to a breakdown product of creatinine phosphate in muscle and is usually produced at a fairly constant rate by the body. The creatinine produced is filtered by the kidney glomeruli and then excreted into the urine without reabsorption.

The expression levels of creatinine can be determined by using any conventional method for protein detection and quantification. In a preferred embodiment, the determination of the expression level of NGAL and the determination of creatinine expression levels is carried out in the same sample, preferably in a urine sample, and at the same time. Thus, the determination of the levels of NGAL in a sample, preferably in a urine sample, can be determined by any of the suitable techniques for protein detection and quantification mentioned above. Creatinine levels can be determined by any suitable technique known by the one skilled in the art. Relevant information about conventional methods for quantifying creatinine in a sample can be found, for example, in Peake et al (Peake M et al., 2006, Clin Biochem Rev, 27: 173-184). By way of non-limiting illustration, said determination can be performed using enzymatic methods using creatinine deaminase, which converts creatinine to N-methlyldantoin and ammonia, or creatininase. It is recommended using an additionally agent to minimize bilirubin protein interference. Examples of said agents include but are not limited to chemical agents such detergents (g.e. sodium duodecil sulphate, litium doudecil sulphate) or potassium ferrycianide or enzymatic agents such bilirubin oxidase. Alternatively, the sample may be filtrated by using an appropriate filter which retains bilirubin (g.e. by using a 30000 MW cut-off filter). Other methods for creatinine quantification which can be used in the present invention are mass spectrometry methods such as gas chromatography coupled to mass spectrometry or liquid chromatography-mass spectrometry. Others common creatinine assays which allow the determination and quantification of creatinine in a sample, preferably in a biofluid such as serum or urine, are the alkaline Jaffe and Benedict-Behre methods (Biol. Chem, 1936, 113: 515) which are run at high pH, typically in the range of from 11.5 to 12.5 in order to deprotonate creatinine, so that the system can operate properly. Other methods which allow the determination of creatinine expression levels in a biofluid, preferably serum or urine, are shown in the patent application numbers US 5804422A and US 5374561A. Adjustment of the pH of the sample, (g.e. urine) is preferable to accommodate the requirements of the protein immunoassay to determine NGAL expression level (e.g. the optimal pH is about 7 to 9). Later on, the pH can be adjusted to 11-5 to 12.5 to determine the creatinine concentration. These methods allow the determination of NGAL and creatinine in the same sample. If desired, the determination of the expression levels of NGAL and creatinine can be run in assays separately, and then the values obtained from these assays can be combined to generate the NGAL/creatinine ratio. Thus, one (or more) of these aliquots can be used for determining NGAL expression level and other (or others) aliquot can be used for determining creatinine levels. It is also possible to determine the expression level NGAL followed by the determination of creatinine expression level in the sample being tested.

Typically the sample is divided in two or more aliquots. In a preferred embodiment, the levels of NGAL and creatinine are expressed in terms of grams (i.e micrograms of NGAL relative to grams of creatinine). If desired, the relative expression level can be expressed in terms of mole % or ratio.

The second step of the first method of the invention comprises comparing the expression level of NGAL obtained in the first step of said method with a reference value. The term "reference value" as used herein, refers to a laboratory value used as a reference for the values/data obtained from samples obtained from the subjects. The reference value (or reference level) can be an absolute value, a relative value, a value which has an upper and/or lower limit, a series of values, an average value, a median, a mean value, or a value expressed by reference to a control or reference value. A reference value can be based on the value obtained from an individual sample, such as, for example, a value obtained from a sample from the subject object of study but obtained at a previous point in time. The reference value can be based on a high number of samples, such as the values obtained in a population of the subjects of the chronological age group coinciding with that of the subject object of study or based on a set of inclusion or exclusion samples of the sample to be analyzed. The reference value can be based on the expression values of the marker to be compared obtained from samples from healthy subjects who do not have a disease state or a particular phenotype. For example, the reference value can be based on the expression level of the marker to be analyzed obtained from subjects who do not have cirrhosis, preferably from healthy subjects without suffering from any liver disease. In a preferred embodiment, the reference value is obtained from a sample or a set of samples from healthy subjects or subjects without prior history cirrhosis. In a particular embodiment, the reference value of NGAL is related to the urine creatinine concentration. As it is shown in the Examples of the present application, values of urine NGAL relative to the urine creatinine concentration are about 35 (10-52) $\mu$g/g of creatinine in healthy subjects. Therefore, if desired, said value can be used as the reference value in the second step of the first method of the invention.

Once the reference value has been established, the expression level of NGAL in the sample from the subject under study is compared with the reference value. As a consequence of this comparison, the expression level of the marker of interest (for example, NGAL in the first method of the invention) in the sample from the subject can be "greater than", "less than" or "equal to" said reference value for said gene. In the context of the present invention, it is considered that an expression level of NGAL in the sample from the subject is "greater than" the reference value for said marker when the expression level of NGAL in the sample from the subject increases, for example, 5%, 10%, 25%, 50%, 100% or even more when compared with the reference value for said gene, or when it increases, for example, at least 1.1-fold, 1.5-fold, 2-fold, 5-fold, 10-fold, 20-fold, 30-fold, 40-fold, 50-fold, 60-fold, 70-fold, 80-fold, 90-fold, 100-fold or even more when compared with the reference value for said marker. In the context of the present invention, it is also considered that an expression level of the marker of interest (for example, NGAL in the first method of the invention) in the sample from the subject is "less than" the reference value for said marker when the expression level of NGAL in the sample from the subject decreases, for example, 5%, 10%, 25%, 50%, 75%, or even 100% when compared with the reference value for said marker.

In the context of the present invention, it is also considered that an expression level of the marker of interest (i.e. NGAL in the first method of the invention) in the sample from the subject is "equal to" the reference value for said marker when the expression level of NGAL is substantially unchanged with respect to the reference value; for example, it is considered that the expression level of NGAL in the sample from the subject under study is "equal to" the reference value when the levels differ by not more than 0.1 %, not more than 0.2%, not more than 0.3%, not more than 0.4%, not more than 0.5%, not more than 0.6%, not more than 0.7%, not more than 0.8%, not more than 0.9%, not more than 1%, not more than 2%, not more than 3%, not more than 4%, not more than 5%, or not more than the percentage value which is the same as the error associated with the experimental method used in the determination.

Once the comparison is made between the expression level of NGAL in the sample from the subject and the reference value for said marker, the first method of the invention allows determining if a subject has high risk of developing ACLF based on if the expression level of NGAL is increased with respect to the reference value.

In a particular embodiment, the patient under study who is being assessed of having a risk of developing ACLF, according to the first method of the invention, shows a high MELD score. The term "MELD score" or "Model for End-Stage Liver Disease" as used herein refers to a scoring system for assessing the severity of chronic liver disease. MELD uses the patient's values for serum bilirubin, serum creatinine and the international ratio for prothrombin time (INR) to predict survival. It is calculated according to the following formula:

$$MELD= 3.78 \, [\text{Ln serum bilirubin (mg/dL)}] + 11.2 \, [\text{Ln INR}] + 9.57 \, [\text{Ln serum creatinine (mg/dL)}] + 6.43$$

wherein, Ln means Napierian logarithm.

The term "bilirubin" as used herein refers to the yellow breakdown product of normal haeme catabolism. Bilirubin is excreted in bile and urine. Most bilirubin (70-90%) is derived from hemoglobin degradation and, to a lesser extent, from other hemo proteins. In serum, bilirubin is usually measured as both direct bilirubin and total-value bilirubin. Direct bilirubin correlates with conjugated bilirubin and it includes both the conjugated bilirubin and bilirubin covalently bound to albumin. Indirect bilirubin correlates to unconjugated bilirubin. The serum bilirubin level can be measured by any suitable method known in the art. Illustrative non-limitative examples of methods for determining serum bilirubin include methods using diazo-reagent, methods with DPD, methods with bilirubin oxidase or by means of direct spectrophotometric determination of bilirubin. Briefly, the method for determining the levels of bilirubin in serum with diazo reagents is based on the formation of azobilirubin, which acts as indicator by means of addition of a mixture of sufanilic acid and sodium nitrite. The method based in determining serum bilirubin with DPD is based on the fact that bilirubin reacts with 2,5-dichlorobenzenediazonium salt (DPD) in 0.1 mol/HCl forming azobilirubin with maximal absorbance at 540-560 nm. The staining intensity is proportional to the concentration of bilirubin. Unconjugated bilirubin reacting in the presence of detergent (e.g. Triton TX-100) is determined as total bilirubin whereas only conjugated bilirubin reacts in the absence of detergent. The method for determining the serum level of bilirubin with bilirubin oxidase is based on the reaction catalized by the enzyme bilirubin oxidase which oxidizes bilirubin to biliverdin with maximal absorbance at 405-460 nm. The concentration of bilirubin is proportional to the measured absorbance. The concentration of total bilirubin is determined by the addition of sodium dodecyl sulfate (SDS) or sodium cholate which evokes the separation of unconjugated bilirubin from albumin and a reaction of precipitation. The level of serum bilirubin can also be determined by direct spectrophotometric at 454 nm and 540 nm. The measurement at these two wavelengths is used to diminish the hemoglobin interference.

The term "international ratio for prothrombin time", or "INR" as used herein, refers to a parameter used to determine the clotting tendency of blood. The INR is the ratio of a patient's prothrombine time to a normal (control) sample, raised to the power of the ISI value for the analytical system used. Prothrombin time (PT) measures factors I (fibrinogen), II (prothrombin), V, VII and X and it is used in conjunction with the activated partial tromboplastin time. The prothrombin time is the time it takes plasma to clot after addition of tissue factor. This measures the extrinsic pathway of coagulation. Normally, in order to measure PT, blood is drawn into a test tube containing an anticoagulant factor, such sodium citrate which acts as anticoagulant by binding the calcium from the sample. Then, the blood sample is centrifuged to obtain the plasma fraction and plasma is treated with an excess of calcium which enables the blood to clot again. Normally, for accurate measurement the proportion of blood to citrate is fixed. Typically, 1 part of anticoagulant to 9 parts to whole blood is used. The INR standardizes the results of prothrombine time and is calculated by the following formula:

$$INR = (PT_{test}/PT_{normal})^{ISI}$$

The ISI value of the formula is the International Sensitive Index for any tissue factor and it indicates how a particular batch of tissue factor compares to an international reference tissue factor. The ISI is usually between 1.0 and 2.0.

The value of MELD score correlates strongly with short-term mortality, the lower the value of MELD score the lower the mortality and the higher the value of the MELD score, the higher the mortality. Thus, a patients having low MELD score, for example a MELD lower than 9 have about 1.9% 3-month mortality whereas patients having high MELD score, for example, a MELD score of 40 or more have about 71.3% 3-month mortality. Relevant information about interpreting the MELD score in hospitalized patients can be found, for example in Wiesner et al. (Wiesner R, et al. Gastroenterology 2003; 124:91-6).

The term "high MELD score" as used herein, refers to a patient having a MELD score higher than 9, for example, at least 10, at least 15, at least 19, at least 20, at least 25, at least 29, at least 30, at least 35, at least 39, at least 40, at least 45 or more. In a still more particular embodiment, the patient to be determined of having a risk of developing ACLF according to the first method of the invention shows a MELD score higher than 20.

In another particular embodiment, the patient to be determined of having a risk of developing ACLF does not show impairment of kidney function. The term "impairment of kidney function", also known as "impairment of renal function", "renal impairment (disorder)", "renal insufficiency", "renal impairment" and "renal failure", as used herein, refers to a medical condition in which the kidneys fail to adequate filter waste products from the blood. Renal failure is mainly determined by a decrease in glomerular filtration rate, the rate which blood is filtered in the glomeruli of the kidney. This is usually detected by a decrease in or absence of urine production or by determination of waste products such as creatinine or urea. In renal failure, there may be problems with increased fluid in the body (leading to swelling), increased acid levels, raised levels of potassium, decreased levels of calcium, increased level of phosphate, and in later stages, anemia. Bone health may also be affected. Two mains forms of renal failure are also included in said definition: acute kidney injury and chronic kidney disease. The term "acute kidney injury" refers to an abrupt loss of kidney function that develops within 48 hours. It is often reversible with adequate treatment and can be due to damage to the glomeruli, renal tubules or interstitium. Common causes of each are glomerulonephritis, acute tubular necrosis and acute interstitial nephritis, respectively. The term "chronic kidney disease" refers to a progressive loss in renal function over a period of months or years which is no reversible.

In a preferred embodiment, the first method of the invention based on the determination of NGAL expression level, further comprises the determination of the expression levels of one or more clinical parameters which are also indicative of liver failure and/or liver disease or are known to be important in these patients. The term "clinical parameter" as used herein, refers to the demographic (illustrative, non-limitative, examples include age and gender) and clinical-pathologic features (illustrative, non-limitative, examples include clinical stage, serum creatinine, and hepatic encephalopathy) that defines the patient, and/or reflects on the manifestation of the disease. Preferably, in the particular case of patients with cirrhosis, suitable clinical markers that can be combined with the first method of the invention include, without limitation, serum bilirubin, leukocyte count, hepatic encephalopathy, MELD score, Child-Pugh score and age.

The term "Child-Pugh score" as used herein, refers to a score used to assess the prognosis as well as the necessity of liver transplantation. The score employs five clinical measures of liver disease: total bilirubin, serum albumin, INR, ascites and hepatic encephalopathy. Each measure is scored 1-3, with 3 indicating most severe derangement. Thus, total bilirubin is scored 1 point if the measurement determines a bilirrubin level of <2 mg/dL; total bilirubin is scored 2 points if the measurement determines a bilirrubin level of 2-3 mg/dL; total bilirubin is scored 3 points if the measurement determines a bilirrubin level of >3 mg/dL. Serum albumin is scored 1 point if the measurement determines an albumin level of >3.5 g/dL; Serum albumin is scored 2 points if the measurement determines an albumin level of 2.8-3.5 g/dL; Serum albumin is scored 3 points if the measurement determines an albumin level <2.8 g/dL. INR is scored 1 point if the measurement determines a INR value of < 1.7; INR is scored 2 points if the measurement determines a INR value of 1.71-2.30; INR is scored 3 points if the measurement determines a INR value of >2.30. Ascites scores 1 point when is not detected; Mild ascites scores 2 points; Moderate to severe ascites scores 3 points. If hepatic encephalopathy is not detected, then scores 1 point; Grade I-II of hepatic encephalopathy scores 2 points; Grade III-IV of hepatic encephalopathy scores 3 points. Chronic liver disease is classified into Child-Pugh class A to C employing the score defined above, wherein if the patient obtains 5-6 points, then is classified as class "A" and the one year-survival probability is about 100% and the two year-survival probability is about 85%; if the patient obtains 7-9 points, then is classified as class "B" and the one year-survival probability is about 81 % and the two year-survival probability is about 57%; if the patient obtains 10-15 points, then is classified as class "C" and the one year-survival probability is about 45% and the two year-survival probability is about 35%.

The term "hepatic encephalopathy" as used herein, refers to the occurrence of confusion, altered level of consciousness and coma as a result of liver failure. In the advanced stages it is called hepatic coma or coma hepaticum. It may ultimately lead to death.

Second method of the invention

The authors of the invention have found that NGAL is a biomarker of prognosis in patients suffering from ACLF and from cirrhosis.

Thus, in another aspect, the invention relates to an *in vitro* method for determining the prognosis of ACLF or cirrhosis or for determining the stage or severity of ACLF or cirrhosis in a subject suffering from said pathology (hereinafter referred as the "second method of the invention") which comprises:

a) determining the expression level of NGAL in a sample from said subject; and
b) comparing the expression level of NGAL obtained in a) with a reference value

wherein if the expression level of NGAL is increased with respect to the reference value, said subject shows bad prognosis of ACLF or cirrhosis or said subject is suffering from advance stage of ACLF.

The term "determining the prognosis" or "prognosis" refers to a prediction of medical outcome, for example, a poor or good outcome (e.g., likelihood of long-term survival, overall survival, disease-specific survival, progression-free survival or disease-free survival); a negative prognosis, or poor outcome, includes a prediction of relapse, disease progression (e.g., advances stages of ACLF), or mortality; a positive prognosis, or good outcome, includes stabilization of the disease. As will be understood by those skilled in the art, the prediction, although preferred to be, need not be correct for 100% of the subjects to be diagnosed or evaluated. The term, however, requires that a statistically significant portion of subjects can be identified as having an increased probability of having a given outcome. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90% at least 95%. The p-values are, preferably 0.05, 0.025, 0.001 or lower.

Any parameter which is widely accepted for determining prognosis of a patient can be used in the present invention including, without limitation: overall survival time, 28-day transplant-free mortality and 90-day transplant-free mortality.

The term "bad prognosis", as used herein, means an outcome which would be regarded negative for the patient and depends on the prognosis type; for example, a bad prognosis of 28-day transplant free mortality would mean that the patient will not survive for at least 28 days without being subjected to a liver transplant. The term "good prognosis", as used herein, means an outcome which would be regarded positive for the patient and depends on the prognosis type; for example, a good prognosis of 28-day transplant-free mortality would mean that the patient will survive for at least 28 days without being subjected to a liver transplant.

The first step of the second method of the invention comprises determining the expression level of NGAL in a sample from said subject. The terms "ACLF", "cirrhosis" "NGAL", "expression level", "sample" and "subject" have been described in detail in the first method of the invention and are equally applicable to the second method of the invention.

In the second step, the second method of the invention comprises comparing the expression level of NGAL obtained in the first step of said method with a reference value.

The reference value (or reference level) can be an absolute value, a relative value, a value which has an upper and/or lower limit, a series of values, an average value, a median, a mean value, or a value expressed by reference to a control or reference value. A reference value can be based on the value obtained from an individual sample or can be based on a high number of samples, such as the values obtained in a population of the subjects of the chronological age group coinciding with that of the subject object of study or based on a set of inclusion or exclusion samples of the sample to be analyzed.

If the second method of the invention is aimed at determining the prognosis of ACLF, then the reference level can be the expression level of NGAL in a subject which suffering ACLF and in which the disease has not further progressed. Progression of the disease can be determined using any of the commonly used end points for ACLF patients, including, transplant-free mortality, 28-day transplant-free mortality, 90-day transplant-free mortality.

If the second method of the invention is aimed at determining the prognosis of cirrhosis, then the reference level can be the expression level of NGAL in a subject which suffering cirrhosis and in which the disease has not further progressed. Progression of the disease can be determined using any of the commonly used end points for ACLF patients, including, transplant-free mortality, 28-day transplant-free mortality, 90-day transplant-free mortality.

If the second method of the invention is aimed at determining the stage of severity of ACLF, then the reference value is the expression level of NGAL in a sample obtained from healthy subjects without prior history of ACLF or from patients having an early stage of ACLF, preferably from patients suffering from grade 1 ACLF.

If the second method of the invention is aimed at determining the stage of severity of cirrhosis, then the reference value is the expression level of NGAL in a sample obtained from healthy subjects without prior history of liver disease or from patients having an early stage of cirrhosis, preferably in patients suffering compensated cirrhosis.

Once this reference value is established, the level of NGAL expressed in samples from subjects can be compared with this reference value, and thus be assigned a level of "increased", "decreased" or "equal". The terms "increased", "decreased" or "equal" have been defined in the context of the first method of the invention and are equally applicable to the second method of the invention.

According to the second method of the invention, if the expression level of NGAL determined in a sample from a subject diagnosed with ACLF is increased with respect to the reference value said subject shows bad prognosis of ACLF.

As it is shown in Example 3 of the present invention, the expression level of NGAL correlates with the severity of ACLF. Thus, the second method of the invention also allows determining the severity of stage of ACLF based on the determination of the expression level of NGAL.

In a particular embodiment, the expression level of NGAL is determined by measuring the mRNA level of the gene encoding NGAL or a fragment thereof or by measuring the amount of protein encoded by NGAL or a variant thereof. Many different approaches may be used for determining the expression level of NGAL. Said methods are described in the context of the first method of the invention and are herein incorporated by reference.

In a particular embodiment, the determination of the expression level of the NGAL protein is carried out by western blot, ELISA or protein array. In a preferred embodiment, the expression level of NGAL is determined by using a commercial ELISA kit (Bioporto, Gentofe, Denmark

In one particular embodiment, the expression level of NGAL is determined in absolute terms, i.e. by providing the concentration of NGAL in a sample. In another particular embodiment, the expression level of NGAL is measured relative to creatinine concentration. In a particular preferred embodiment, the expression level of NGAL is measured relative to urine creatinine concentration. Many different approaches may be used for determining the levels of creatinine. Said methods are described in the context of the first method of the invention.

In a preferred embodiment, the patient suffering from ACLF whose prognosis is determined by means of 28-day transplant-free mortality is classified as having good prognosis if the NGAL value relative to creatinine concentration is lower than 86.5μg/g. On the other hand, if the prognosis is determined by means of 28-day transplant-free mortality, the patient is classified as having bad prognosis if the NGAL value relative to creatinine concentration is higher than 86μg/g.

In another preferred embodiment, the patient suffering from ACLF whose prognosis is determined by means of 90-day transplant-free mortality is classified as having good prognosis if the NGAL value relative to creatinine concentration is lower than 65.9μg/g. On the other hand, if the prognosis is determined by means of 28-day transplant-free mortality, the patient is classified as having bad prognosis if the NGAL value relative to creatinine concentration is higher than 66/μg/g.

In a particular embodiment, said sample is a biofluid selected from urine or serum. In a preferred embodiment of the present invention, said biofluid is urine.

In a particular embodiment, the second method of the invention refers to an *in vitro* method for determining the prognosis of ACLF, or for determining the stage of severity of ACLF in a subject suffering from said pathology, wherein ACLF is stage 1. In another particular embodiment, the second method of the invention refers to an *in vitro* method for determining the prognosis ACLF, or for determining the stage of severity of ACLF in a subject suffering from said pathology, wherein ACLF is stage 2. In another particular embodiment, the second method of the invention refers to an *in vitro* method for determining the prognosis of ACLF, or for determining the stage of severity of ACLF in a subject suffering from said pathology, wherein ACLF is stage 3. The terms "stage 1 of ACLF"," stage 2 of ACLF" and "stage 3 of ACLF" have been defined in the context of the first method of the invention and are equally applicable to the second method of the invention.

According to the second method of the invention, if the expression level of NGAL determined in a sample from a subject diagnosed with cirrhosis is increased with respect to the reference value said subject shows bad prognosis of cirrhosis.

In the context of the present invention, it is considered that an expression level of NGAL in the sample from the subject is increased with respect to the reference value for said marker when the expression levels of NGAL in the sample are for example, 5%, 10%, 25%, 50%, 100% or even higher than the levels in the reference value for said gene, or when it increases, for example, at least 1.1-fold, 1.5-fold, 2-fold, 5-fold, 10-fold, 20-fold, 30-fold, 40-fold, 50-fold, 60-fold, 70-fold, 80-fold, 90-fold, 100-fold or even more when compared with the reference value for said marker. In the context of the present invention, it is also considered that an expression level of the marker of interest (for example, NGAL in the second method of the invention) in the sample from the subject is "less than" the reference value for said marker when the expression level of NGAL in the sample from the subject decreases, for example, 5%, 10%, 25%, 50%, 75%, or even 100% when compared with the reference value for said marker.

In a particular embodiment, the second method of the invention refers to an *in vitro* method for determining the prognosis of cirrhosis, or for determining the stage of severity of cirrhosis in a subject suffering from said pathology, wherein the cirrhosis is decompensated. The term "decompensated cirrhosis" has been described in detail in the first method of the invention and is equally applicable to the second method of the invention.

In a particular preferred embodiment, the prognosis is determined by a parameter selected from the group consisting of survival time, 28-day transplant-free mortality and 90-day transplant-free mortality.

The term "survival time" or "overall survival time", as used herewith, relates to the percentage of people in a study or treatment group who are alive for a certain period of time after they were diagnosed with or treated for a disease, such as ACLF or cirrhosis.

The term "28-day transplant free mortality", as used herein, is defined as the percentage of subjects who are alive in the first 28 days without being subjected to a liver transplant after a given event in the course of the disease, such as the diagnosis of a complication or the development of ACLF or cirrhosis, the diagnosis of a life-threatening complications associated with ACLF or cirrhosis or the initiation of the hospitalization.

The term "90-day transplant free mortality", as used herein, is understood as the percentage of subjects who are alive in the first 90 days without being subjected to a liver transplant after they were diagnosed from a specific complication of cirrhosis or development of ACLF or after the initiation of the hospitalization

In a particular embodiment, the patient under study who is being assessed of having a risk of developing ACLF, according to the first method of the invention, shows a high MELD score. The term "MELD score" has been described in detail in the first method of the invention and is equally applicable to the second method of the invention. In a still more preferred embodiment of the second method of the invention, the patient shows a MELD score higher than 20.

In another particular embodiment of the second method of the invention, the patient does not show impairment of kidney function. The term "impairment of kidney function" has been described in detail in the first method of the invention and is equally applicable to the second method of the invention.

In a preferred embodiment, the second method of the invention based on the determination of NGAL expression level, further comprises the determination of the expression levels of one or more clinical parameters which are also indicative of liver failure and/or liver disease or are known to be important in these patients. Suitable clinical markers that can be combined with the second method of the invention include, without limitation, serum bilirubin, leukocyte count, hepatic encephalopathy, MELD score, Child-Pugh score and age.

Third method of the invention

In another aspect, the invention relates to an *in vitro* method for monitoring the effect of a therapy in a patient suffering from ACLF and being treated with said therapy which comprises:

a) determining the expression level of NGAL in a sample from said subject; and
b) comparing the expression level of NGAL obtained in a) with a the expression level of NGAL in a sample from said subject at earlier point of time,

wherein if the expression level of NGAL is decreased with respect to the expression level of NGAL determined in a sample from said subject at earlier point of time the therapy is being effective.

The term "therapy", as used herein, refers to the attempted remediation of a health problem, usually following a diagnosis, or to prevention or the appearance of a health problem. As such, it is not necessarily a cure, i.e. a complete reversion of a disease. Said therapy may or may not be known to have a positive effect on a particular disease. This term includes both therapeutic treatment and prophylactic or preventative measures, in which the object is to prevent or stop (reduce) an undesired physiological change or disorder, such as, ACLF. For the purpose of this invention, beneficial or desired clinical results include, without limitation, relieving symptoms, stabilizing pathological state (specifically not worsening), slowing down or stopping the progression of the disease, improving or mitigating the pathological impairment. Particularly, for the purpose of the present invention, therapy is directed to slow the progression of liver damage and reduce the risk of further complications. It can also involve prolonging survival in comparison with the expected survival if treatment is not received. Those subjects needing treatment include those subjects already suffering the condition or disorder, as well as those with the tendency to suffer the condition or disorder or those in which the condition or disorder must be prevented.

The term "treatment", as used herein, relates to both therapeutic measures and prophylactic or preventative measures, wherein the object is to prevent or slow down (lessen) an undesired physiological change or disorder, such as ACLF. Beneficial or desired clinical results include, but are not limited to, alleviation of symptoms, stabilizing pathological state (specifically not worsening), slowing down or stopping the progression of the disease, improving or mitigating the pathological. Particularly, for the purpose of the present invention, treatment is directed to slow the progression of liver damage and reduce the risk of further complications.

The terms "ACLF", "NGAL", "expression level", "sample" and "subject" have been described in detail in the first method of the invention and are equally applicable to the third method of the invention.

According to this inventive aspect, the expression level of NGAL determined in a sample from a subject having ACLF obtained at a first time (first subject sample) and the expression level of NGAL determined in the sample from said subject at second period of time (second subject sample) are compared allowing the monitorization of the effect of a therapy in said subject having ACLF. The second subjects sample can be taken at any time after the first period of time, e.g., one day, one week, two weeks, three weeks, one month, two month, three months or more after the first subject sample. In a particular embodiment, the first sample is taken after the subject has started received treatment for said clinical condition. Therapies which can be used to treat a patient suffering from ACLF are focused to treat the liver failure and the end-organ dysfunction associated to said condition. Examples of said therapies include but are not limited to therapies directed to attenuate the immune response, such as pentoxifylline, therapies to prevent translocation of aerobic bacteria from the gut, such as norfloxacin or rifaximin, therapies to treat portal hypertension and portal hypertensin related complications (g.e. variceal bleeding) such as somatostain or terlipressin, therapies to treat bacterial infection, including systemic administration of antibiotics, therapies to treat circulatory dysfunction, including the administration of vasopressor agents, therapies to treat hepatic encephalopathy including lactulose, lactitol, and cleansing enemas, therapies to treat respiratory failure, including oxygen administration or assisted ventilation, therapies to treat kidney failure, including hemodyalisis or filtration therapies, therapies to treat liver failure including the so-called liver-assisted devices such as MARS or Prometheus or plasmapheresis or any other therapy aimed at improving the effects of liver failure. The effectiveness of the therapy after the treatment can be easily followed according to the teachings of this invention.

Thus, according to the third method of the invention, if the expression level of NGAL is decreased with respect to the expression level of NGAL determined in a sample from said patient at earlier point of time the therapy is being effective. On the contrary, if no decrease or increase, that is to say if the expression level of NGAL has the same value or is increased with respect to the expression level of NGAL in the first subject sample is achieved, then the therapy is not being effective.

In a particular embodiment, said sample is a biofluid selected from urine or serum. In a preferred embodiment of the present invention, said biofluid is urine.

In a particular embodiment, the expression level of NGAL can be determined by measuring the mRNA level of the gene encoding NGAL or a fragment thereof or by measuring the amount of protein encoded by NGAL or a variant thereof. Many different approaches may be used for determining the expression level of NGAL. Said methods are described in the context of the first method of the invention are herein incorporated by reference.

In a particular embodiment, the determination of the expression level of the NGAL protein is carried out by western blot, ELISA or protein array. In a preferred embodiment, the expression level of NGAL is determined by using a commercial ELISA kit (Bioporto, Gentofe, Denmark).

In one particular embodiment, the expression level of NGAL is determined in absolute terms, i.e. by providing the concentration of NGAL in a sample. In another particular embodiment, the expression level of NGAL is measured relative to creatinine concentration. In a particular preferred embodiment, the expression level of NGAL is measured relative to urine creatinine concentration. Many different approaches may be used for determining the levels of creatinine. Said methods are described in the context of the first method of the invention.

In a particular embodiment, the patient under study, according to the third method of the invention, shows a high MELD score. The term "MELD score" has been described in detail in the first method of the invention and is equally applicable to the third method of the invention. In a still more preferred embodiment of the third method of the invention, the patient shows a MELD score higher than 20.

In another particular embodiment of the third method of the invention, the patient does not show impairment of kidney function. The term "impairment of kidney function" has been described in detail in the first method of the invention and is equally applicable to the third method of the invention.

In a preferred embodiment, the third method of the invention based on the determination of NGAL expression level, further comprises the determination of the expression levels of one or more clinical parameters which are also indicative of liver failure and/or liver disease or are known to be important in these patients. Suitable clinical markers that can be combined with the third method of the invention include, without limitation, serum bilirubin, leukocyte count, hepatic encephalopathy, MELD score, Child-Pugh score and age.

Fourth method of the invention

The authors of the present invention have found that the expression level of NGAL is higher in patients suffering from ACLF than in patients having acute decompensation of cirrhosis.

Thus, in another aspect, the invention relates to *an in vitro* method for the differential diagnoses acute-on chronic-liver failure (ACLF) from acute decompensation of cirrhosis in a subject (hereinafter, "the fourth method of the invention") which comprises:

a) determining the expression level of NGAL in a sample from said subject; and
b) comparing the expression level of NGAL obtained in a) with a reference value

wherein if the expression level of NGAL is increased with respect to the reference value, said subject suffers ACLF.

The term "differential diagnoses" or "differentially diagnosing", as used herein, relates to the determination of a different condition. As will be understood by those skilled in the art, differentiation, although preferred to be, need not be correct for 100% of the subjects to be diagnosed or evaluated. The term, however, requires that a statistically significant portion of subjects can be identified as having an increased probability of having one of the two types of condition, i.e., ACLF or acute decompensation of cirrhosis. Whether a subject is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.05, 0.01, 0.005 or lower.

The terms "ACLF", "acute decompensated cirrhosis" "NGAL", "expression level", "sample" and "subject" have been described in detail in the first method of the invention and are equally applicable to the fourth method of the invention.

According to the fourth method of the invention, after comparing the results obtained in the sample from the individual under study with the results of the reference value, an increase in the expression level of NGAL in the sample from said individual with respect to the expression level of NGAL in the reference value is indicative of ACLF.

In a particular embodiment, said sample is a biofluid selected from urine or serum. In a preferred embodiment of the present invention, said biofluid is urine.

In a particular embodiment, the expression level of NGAL can be determined by measuring the mRNA level of the gene encoding NGAL or a fragment thereof or by measuring the amount of protein encoded by NGAL or a variant thereof. Many different approaches may be used for determining the expression level of NGAL. Said methods are described in the context of the first method of the invention and are herein incorporated by reference.

In a particular embodiment, the determination of the expression level of the NGAL protein is carried out by western blot, ELISA or protein array. In a preferred embodiment, the expression level of NGAL is determined by using a commercial ELISA kit (Bioporto, Gentofe, Denmark).

In one particular embodiment, the expression level of NGAL is determined in absolute terms, i.e. by providing the concentration of NGAL in a sample. In another particular embodiment, the expression level of NGAL is measured relative to creatinine concentration. In a particular preferred embodiment, the expression level of NGAL is measured relative to urine creatinine concentration. Many different approaches may be used for determining the levels of creatinine. Said methods are described in the context of the first method of the invention.

In a preferred embodiment of the invention, the subject is diagnosed as having ACLF if the expression level of NGAL is higher than 50 $\mu$g NGAL/g creatinine.

In a particular embodiment, the patient under study who is being assessed of having ACLF or acute decompensation of cirrhosis, according to the fourth method of the invention, shows a high MELD score. The term "MELD score" has been described in detail in the first method of the invention and is equally applicable to the fourth method of the invention. In a still more preferred embodiment of the fourth method of the invention, the patient shows a MELD score higher than 20.

In another particular embodiment, the patient does not show impairment of kidney function. The term "impairment of kidney function" has been described in detail in the first method of the invention and is equally applicable to the fourth method of the invention.

In a preferred embodiment, the fourth method of the invention based on the determination of NGAL expression level, further comprises the determination of the expression levels of one or more clinical parameters which are also indicative of liver failure and/or liver disease or are known to be important in these patients. Suitable clinical markers that can be combined with the fourth method of the invention include, without limitation, serum bilirubin, leukocyte count, hepatic encephalopathy, MELD score, Child-Pugh score and age.

Fifth method of the invention

In another aspect, the invention relates to an *in vitro* method for selecting a patient suffering from ACLF for a therapy aimed at treating the effects of liver failure and/or for selecting a patient for liver transplantation (hereinafter, "the fifth method of the invention") which comprises:

a) determining the expression level of NGAL in a sample from said patient; and
b) comparing the expression level of NGAL obtained in a) with a reference sample

wherein if the expression level of NGAL is increased with respect to the reference sample said patient is a candidate for receiving a therapy aimed at treating the effects of liver failure and/or for liver transplantation.

In one embodiment, the fifth aspect of the invention refers to a method for selecting a patient suffering from ACLF for a therapy aimed at treating the effects of liver failure. The term "selecting a patient for a therapy", as used herein, relates to the identification of a patient for a therapy designed to cure a disease or palliate the symptoms associated with one or more diseases or conditions. In the particular case of ACLF therapy, it is understood any therapy which abolishes, retards or reduces the symptoms associated with liver failure. Details of adequate therapies which can be used according to the invention to treat ACLF have been explained in the context of the third aspect of the invention and are herein incorporated by reference.

As will be understood by those skilled in the art, the selection of a patient, although preferred to be, need not be adequate for 100% of the subjects selected according to the fifth method of the invention. The term, however, requires that a statistically significant portion of subjects were correctly selected. Whether the selection of a patient in a population of subjects is statistically significant can be determined without further ado by the person skilled in the art using various well known statistic evaluation tools, e.g., determination of confidence intervals, p-value determination, Student's t-test, Mann-Whitney test, etc. Details are found in Dowdy and Wearden, Statistics for Research, John Wiley & Sons, New York 1983. Preferred confidence intervals are at least 50%, at least 60%, at least 70%, at least 80%, at least 90%, or at least 95%. The p-values are, preferably, 0.2, 0.1 or 0.05.

In another embodiment, the fifth aspect of the invention refers to a method for selecting a patient suffering from ACLF for liver transplantation. The term "liver transplantation" as used herein, refers to the replacement of a diseased liver with some or all of a healthy liver from another person. The most commonly used technique is orthotopic transplantation, in which the native liver is removed and replaced by a liver from an organ donor in the same anatomic location as the original liver.

The terms "ACLF", "acute decompensated cirrhosis" "NGAL", "expression level", "sample" and "subject" have been described in detail in the first, second, third and fourth method of the invention and are equally applicable to the fifth method of the invention.

According to the fifth method of the invention, after comparing the results obtained in the sample from the individual under study with the results of the reference value, an increase in the expression level of NGAL in the sample from said individual with respect to the expression level of NGAL in the reference value is indicative of that the patient is a candidate for receiving therapy aimed at treating the effects of liver failure and/or for liver transplantation.

In a particular embodiment, said sample is a biofluid selected from urine or serum. In a preferred embodiment of the present invention, said biofluid is urine.

In a particular embodiment, the expression level of NGAL can be determined by measuring the mRNA level of the gene encoding NGAL or a fragment thereof or by measuring the amount of protein encoded by NGAL or a variant thereof. Many different approaches may be used for determining the expression level of NGAL. Said methods are described in the context of the first method of the invention and are herein incorporated by reference.

In a particular embodiment, the determination of the expression level of the NGAL protein is carried out by western blot, ELISA or protein array. In a preferred embodiment, the expression level of NGAL is determined by using a commercial ELISA kit (Bioporto, Gentofe, Denmark).

In one particular embodiment, the expression level of NGAL is determined in absolute terms, i.e. by providing the concentration of NGAL in a sample. In another particular embodiment, the expression level of NGAL is measured relative to creatinine concentration. In a particular preferred embodiment, the expression level of NGAL is measured relative to urine creatinine concentration. Many different approaches may be used for determining the levels of creatinine. Said methods are described in the context of the first method of the invention.

In a particular embodiment, the patient under study, according to the fifth method of the invention, shows a high MELD score. The term "MELD score" has been described in detail in the first method of the invention and is equally applicable to the fifth method of the invention. In a still more preferred embodiment of the fifth method of the invention, the patient shows a MELD score higher than 20.

In another particular embodiment of the fifth method of the invention, the patient does not show impairment of kidney function. The term "impairment of kidney function" has been described in detail in the first method of the invention and is equally applicable to the fifth method of the invention.

In a preferred embodiment, the fifth method of the invention based on the determination of NGAL expression level, further comprises the determination of the expression levels of one or more clinical parameters which are also indicative of liver failure and/or liver disease. Suitable clinical markers that can be combined with the fifth method of the invention include, without limitation, serum bilirubin, leukocyte count, hepatic encephalopathy, MELD score, Child-Pugh score and age.

Uses of the invention

In another aspect, the invention relates to the use of NGAL for determining the risk of developing ACLF in a subject suffering from cirrhosis.

In another aspect, the invention relates to the use of NGAL for determining the prognosis of a patient suffering from ACLF.

In another aspect, the invention relates to the use of NGAL for determining the prognosis of a patient suffering from cirrhosis.

In another aspect, the invention relates to the use of NGAL for determining the stage or severity of a patient suffering from ACLF.

In another aspect, the invention relates to the use of NGAL for determining the stage or severity of a patient suffering from cirrhosis.

In another aspect, the invention relates to the use of NGAL for monitoring the effect of a therapy in a patient suffering from ACLF.

In another aspect, the invention relates to the use of NGAL for selecting a patient suffering from ACLF for a therapy aimed at treating the effects of liver failure and/or for liver transplantation.

In another aspect the invention relates to the use of NGAL for the differential diagnosis of ACLF from acute decompensation of cirrhosis.

The terms "NGAL", "determining the risk", "ACLF", "cirrhosis", "prognosis", "therapy", "subject", "acute decompensation of cirrhosis" and "differential diagnosis" have been defined in the context of the first, second, third, fourth and fifth method of the invention and are equally applicable to the uses of the invention.

The following examples serve to illustrate the invention and must not be considered as limiting the scope thereof. To carry out said examples, the materials and methods described therein were used.

**Examples**

**Materials and Methods**

Study population

The current study was performed in a population of patients included in the CANONIC study (Moreau et al, Gastroenterology 2013;144:1426-37). The CANONIC study was a multicentre investigation which included 1,343 patients admitted for an acute decompensation of cirrhosis aimed at evaluating the frequency, characteristics, and outcome of acute-on-chronic-liver failure (ACLF). The study was performed in 29 hospitals from 9 nine European countries. In 716 of the 1,343 patients (53.3%), a urine sample was collected at the time of admission into the study, centrifuged at 1,000 rpm and the supernatant stored at -80°C for later measurement of urine levels of neutrophil gelatinase-associated lipocalin (NGAL). In the remaining patients urine could not be collected because of logistical reasons. The 716 patients with urine samples constitute the population of the current study. Patients with urine samples had similar baseline characteristics compared to those of patients who did not have a urine sample collected.

The baseline characteristics of the study group are shown in table 1. As expected, patients had advanced cirrhosis as indicated by marked impairment of liver function tests and high Child-Pugh and MELD scores. The latter are the most commonly used prognostic scores to assess disease severity and predict outcome of patients with cirrhosis *(Kamath PS et al. Hepatology 2007;445:797-805; Durand F and Valla D. Semin Liver2008;28:110-22)*

**Table 1.** Baseline characteristics of patients included at the time of admission in the study.

| | |
|---|---|
| Age (years) | 58±12 |
| Sex (male/female) | 467/249 |
| Etiology of cirrhosis Alcohol / Other | 323/393 |
| Serum bilirubin (mg/dL) | 5.7±7.2 |
| Serum albumin (g/L) | 29±9 |
| INR (units) | 1.6±0.6 |
| Serum creatinine (mg/dL) | 1.3±0.9 |
| Serum sodium (mEq/L) | 135±6 |
| Leukocyte count (x $\mu$L) | 7.2±4.6 |
| C-reactive protein (mg/L) | 28.4±32.8 |
| MELD score | 18.3±7.0 |
| Child-Pugh score | 9.5±2.1 |

Main variables and definitions

ACLF is a complication of patients with cirrhosis that is characterized by the presence of organ failure(s) (either hepatic or extrahepatic) and entails a poor short-term prognosis *(Moreau R, et al. Gastroenterology 2013;144:1426-37)*. ACLF was defined according to the presence and severity of organ failures as described in the CANONIC study. Patients with ACLF were classified in 3 different grades according to disease severity: 1/ ACLF grade 1; 2/ ACLF grade 2; and 3/ ACLF grade 3.
Outcome was analysed as 28-day and 90-day transplant-free mortality.

NGAL measurement

The urinary concentration of NGAL was measured using an ELISA kit (Bioporto, Gentofte, Denmark). Coefficients of inter-assay variation were 12.6% and 3.4%, respectively. NGAL was reported relative to the urine creatinine concentration. Normal values of uNGAL in healthy subjects are 35 (10-52) $\mu$g/g of creatinine (median and IQ range).

Statistical analysis

Data were collected using an electronic case report form. Quantitative variables are reported as median and interquartile range or mean (±SD). Categorical variables are reported as count and percentage. Correlations between quantitative variables were performed with Spearman coefficient. Factors associated with 28-day or 90-day transplant-free mortality in all series as well as in the subgroup of patients with or without ACLF were identified in a bivariate analysis with Student's T-test and one-way ANOVA for quantitative variables (depending of the number of categories), or the non-parametric corresponding tests, if needed. Twenty-eight-day and 90-day mortality rates were estimated as transplant-free mortality. Multivariate logistic regression models were fitted to select main factors independently associated with the endpoint. A forward stepwise procedure (variable entry/drop criteria, $p<0.05$ / $p>0.1$) was applied to select factors significantly contributing to model fit. The accuracy of the models in predicting 28-day and 90-day transplantation-free mortality was assessed estimating the Area Under the Curve of the Receiving Operating Characteristic (AUCROC). The significance level for all statistical tests was set at 0.05 two-tailed. All statistical analyses were performed using SAS 9.2 software.

**Example 1: uNGAL levels and their relationship with clinical and laboratory variables**

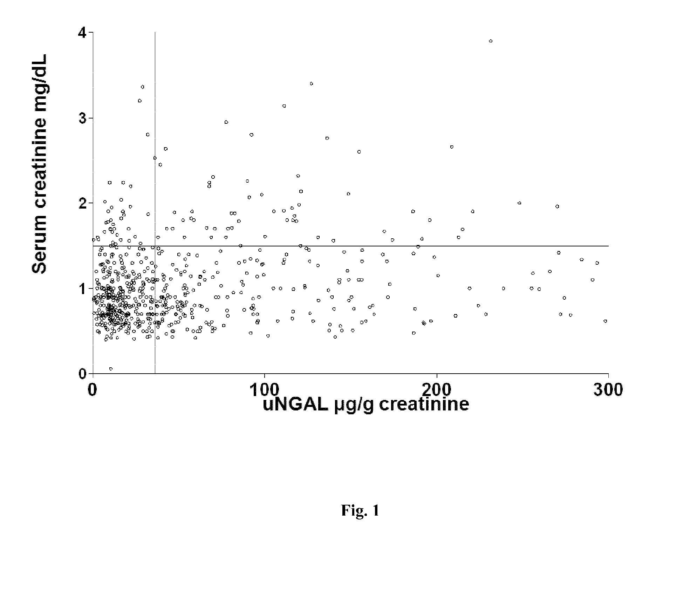

In the overall series uNGAL levels were 207±960 $\mu$g/g of creatinine, a value strikingly higher than the average value in healthy subjects. Among a large number of quantitative baseline variables analyzed, uNGAL correlated with some variables, including age, serum bilirubin, international normalized ratio (INR), leukocyte count, and C-reactive protein, but correlations were weak (Spearman coefficients, 0,15, 0.09, 0.09, 0.23, and 0.25, respectively). Considering that NGAL is overexpressed in the kidneys and that uNGAL may be increased in the context of an impairment of kidney function, we sought to specifically analyze the relationship between uNGAL and serum creatinine. Overall, the correlation between uNGAL and serum creatinine was poor, yet statistically significant (r=0.26; p<0.001). Figure 1 is a plot of the individual values of uNGAL and serum creatinine levels in all patients included in the study categorized in four goups according to the median value of uNGAL and a serum creatinine value of 1.5 mg/dL, which is the most common cut-off level used to define kidney failure in cirrhosis (Arroyo et al. Hepatology 1996; 23:164-76; Ginès P and Schrier RW. New England J Med 2009; 361:1279-90). As shown in figure 1, a substantial number of patients (257 out of the 716 - 35.9%) had high values of uNGAL in the setting of low serum creatinine values. On the other hand, there were patients with high serum creatinine values who had low uNGAL levels. Therefore, these data suggest that in cirrhosis increased uNGAL cannot be explained merely by an impairment of kidney function.

uNGAL also correlated with some qualitative variables, including sex, presence of bacterial infection or hepatic encephalopathy and absence of gastrointestinal hemorrhage (median values in females, patients with bacterial infection, hepatic encephalopathy or without gastrointestinal bleeding were 69 (33-170), 44 (20-157), 53 (21-170), and 41 (16-116) $\mu$g/g of creatinine, respectively; corresponding values in their counterpart groups were 25 (11-66), 34 (13-96), 32 (13-87), and 24 (11-55) respectively; p<0.01 for all).

**Example 2: Relationship between uNGAL levels and 28-day and 90-day transplant-free mortality in the overall series of patients**

Within a 28-day period after admission, 57 (7.9%) patients died, 633 (88.4%) were alive, and 26 (3.6%) were transplanted. Corresponding values for a 90-day period were 119 (16.9%), 521 (74.1%), and 63 (8.9%), respectively. In univariate analysis, variables obtained at admission of patients in the study that were associated with 28-day transplant-free mortality were: presence of ascites, hepatic encephalopathy, or bacterial infections, serum bilirubin, leukocyte count, INR, serum creatinine, and C-reactive protein, Child-Pugh and MELD scores, and uNGAL. Median uNGAL values in patients who died were markedly higher than in those who survived (204 (87-1314) vs 33 (13-88) $\mu$g/g of creatinine, respectively; p<0.0001). Tables 2 and 3 show the multivariate models of 28-day transplant-free mortality including only the individual laboratory and clinical variables which were statistically significant in the univariate analyses (table 2) or these variables plus the prognostic scores (table 3).

**Table 2.** Multivariate model of 28-day transplant-free mortality in the overall series. The model includes only individual clinical and laboratory variables.

| Variable | OR | IQR |
|---|---|---|
| **uNGAL*** | **2.09** | **1.66-2.63** |
| **Serum bilirubin*** | **2.17** | **1.56-3.02** |
| **Leukocyte count*** | **2.29** | **1.29-4.09** |
| **Hepatic encephalopathy** | **2.09** | **1.08-4.07** |
| *log transformed        AUCROC: 0.886 (0.84-0.93) | | |

**Table 3.** Multivariate model of 28-day transplant-free mortality in the overall series. The multivariate analysis was performed including individual clinical and laboratory variables as well as prognostic scores.

| Variable | OR | IQR |
|---|---|---|
| **uNGAL*** | **1.77** | **1.42-2.21** |
| **MELD score** | **1.12** | **1.07-1.17** |
| **Leukocyte count*** | **2.16** | **1.26-3.72** |
| *log transformed        AUCROC: 0.876 (0.83-0.92) | | |

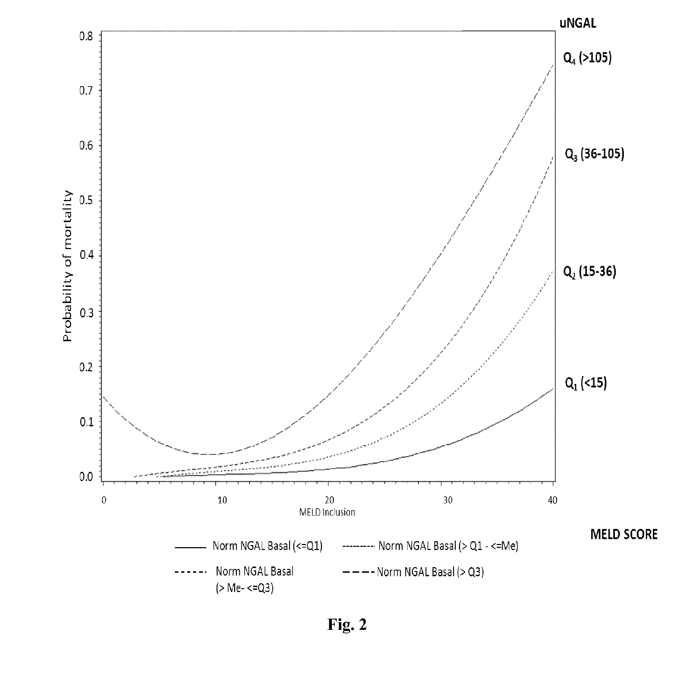

As shown in multivariate models of tables 2 and 3, independent predictive factors of 28-day transplant-free mortality were uNGAL, serum bilirubin, leukocyte count, and hepatic encephalopathy (model 1) and uNGAL, MELD score, and leukocyte count, respectively (model 2). Remarkably, uNGAL was an independent predictive factor of short-term mortality in both models. Moreover, and most importantly, uNGAL improved the prognostic accuracy of MELD score. The MELD score is a prognostic scoring system that uses a combination of three variables, serum bilirubin, serum creatinine, and INR that is the most widespread and frequently used scoring system to assess the severity of cirrhosis and evaluate short-term mortality in these patients (Kamath PS et al. Hepatology 2007; 45: 797-805; Durand F, Valla D. Semin Liver Dis 2008;28:110-22). Given its high accuracy, MELD score has been adopted by transplant organizations of many countries as the method for organ allocation to patients in the waiting list for liver transplantation. To further evaluate the added value of uNGAL to the MELD score, we assessed the relationship between MELD score and 28-day probability of transplant-free mortality in patients included in the study according to the levels of uNGAL. As shown in figure 2, the probability of mortality was markedly influenced by the levels of uNGAL, particularly at values of MELD score greater than 20. Patients with high MELD scores but low uNGAL values had low 28-day transplant-free mortality, while patients with high MELD scores but high uNGAL values had very high 28-day transplant-free mortality.

Similarly to 28-day transplant-free mortality, uNGAL was also an independent predictive factor of 90-day transplant-free mortality, even if prognostic scores were included in the multivariate analysis (table 4). Moreover, alike to 28-day transplant-free mortality uNGAL levels improved the accuracy of MELD score in the prediction of 90-day transplant-free mortality (figure not shown).

**Table 4.** Multivariate model of 90-day transplant-free mortality in the overall series. The multivariate analysis was performed including individual clinical and laboratory variables as well as prognostic scores.

| Variable | OR | IQR |
|---|---|---|
| uNGAL* | 1.44 | 1.21-1.71 |
| MELD | 1.18 | 1.13-1.23 |
| Age | 1.04 | 1.02-1.06 |
| Leukocyte count* | 2.17 | 1.39-3.36 |
| *log transformed  Area-under-the -ROC curve (AUC): 0.856 (0.82-0.89) | | |

## Example 3: Relationship between uNGAL levels and acute-on-chronic liver failure

We next examined whether uNGAL could be of value as biomarker of prognosis in patients with ACLF or as predictive factor of ACLF development in patients without ACLF. Currently, there are no biomarkers of ACLF development or prognosis.

At admission, 148 (20.7%) patients had ACLF, while the remaining 568 patients had classical acute decompensation of cirrhosis without ACLF. Eighty-four patients had ACLF grade 1, 52 grade 2, and 12 grade 3. Patients with ACLF had significantly higher values of uNGAL compared to those of patients without ACLF (108 (35-400) vs 29 (12-73) $\mu$g/g of creatinine, respectively; p<0.0001). Moreover, uNGAL levels increased progressively with the severity of ACLF (79 (29-281), 108 (40-261), and 2,458 (245-3,280) $\mu$g/g of creatinine in patients with ACLF grade 1, 2, and 3, respectively; p<0.0001). In multivariate analysis, the only independent predictive factors of 28-day and 90-day transplant-free mortality in patients with ACLF were uNGAL and MELD score. None of the remaining clinical and analytical variables or prognostic scores had independent prognostic value in patients with ACLF.

Sixty of the 568 patients (10.6%) without ACLF at admission developed ACLF during hospitalization. uNGAL levels measured at the time of hospital admission in patients without ACLF had independent predictive value of ACLF development (OR 1.37 (1.11-1.70). Taken together, these results indicate that uNGAL is a biomarker of short-term prognosis in patients with ACLF and also a biomarker of prediction of ACLF development in patients admitted to hospital for an acute decompensation of cirrhosis.

## Example 4: Relationship between uNGAL levels and 28-day and 90-day mortality in patients with cirrhosis without ACLF

Out of the 568 patients without ACLF at admission, 21 (3.7%) patients died, 530 (93.3%) were alive, and 17 (3.0%) had been transplanted within a 28-day period. Corresponding values for a 90-day period were 63 (11.1%), 448 (78.9%), and 45 (7.9%) (an additional 12 patients were lost to follow-up), respectively. Tables 5 and 6 show the multivariate models of 28-day transplant-free mortality and 90-day transplant-free mortality in these patients. At both time points, uNGAL levels had independent predictive value of transplant-free mortality of patients with cirrhosis without ACLF.

**Table 5.** Multivariate model of 28-day transplant-free mortality in patients with cirrhosis without ACLF. The multivariate analysis was performed including individual clinical and laboratory variables as well as prognostic scores.

| Variable | OR | IQR |
|---|---|---|
| uNGAL* | 1.61 | 1.15-2.25 |
| Leukocyte count* | 3.25 | 1.46-7.27 |
| *log transformed        AUCROC: 0.778 (0.69-0.87) | | |

**Table 6.** Multivariate model of 90-day transplant-free mortality in patients with cirrhosis without ACLF. The multivariate analysis was performed including individual clinical and laboratory variables as well as prognostic scores.

| Variable | OR | IQR |
|---|---|---|
| MELD | 1.18 | 1.11-1.26 |
| uNGAL* | 1.47 | 1.17-1.84 |
| Leukocyte count* | 2.31 | 1.34-3.98 |
| Age | 1.04 | 1.01-1.06 |
| *log transformed        Area-under-the -ROC curve (AUC): 0.816 (0.76-0.87) | | |

**Claims**

1.  An *in vitro* method for determining the risk of developing acute-on chronic liver-failure (ACLF) in a subject suffering from cirrhosis which comprises:

    a) determining the expression level of Neutrophil Gelatinase-Associated Lipocalin (NGAL) in a sample from said subject; and
    b) comparing the expression level of NGAL obtained in a) with a reference value

    wherein if the expression level of NGAL is increased with respect to the reference value said subject has high risk of developing ACLF.

2.  An *in vitro* method for determining the prognosis of ACLF or cirrhosis or for determining the stage or severity of ACLF or cirrhosis in a subject suffering from said pathology which comprises:

    a) determining the expression level of NGAL in a sample from said subject; and
    b) comparing the expression level of NGAL obtained in a) with a reference value

    wherein if the expression level of NGAL is increased with respect to the reference value, said subject shows bad prognosis of ACLF or cirrhosis or said subject is suffering from advanced stage of ACLF or cirrhosis.

3.  The method according to claim 1 or 2, wherein the cirrhosis is decompensated.

4.  The method according to claims 2 or 3, wherein the prognosis is determined by a parameter selected from the group consisting of survival time, 28-day transplant free mortality and 90-day transplant free mortality.

5.  The method according to any of claims 2 to 4, wherein ACLF is stage 1, stage 2 or stage 3.

6.  An *in vitro* method for monitoring the effect of a therapy in a patient suffering from ACLF and being treated with said therapy which comprises:

    a) determining the expression level of NGAL in a sample from said patient; and
    b) comparing the expression level of NGAL obtained in a) with a the expression level of NGAL in a sample from said patient at earlier point of time,

wherein if the expression level of NGAL is decreased with respect to the expression level of NGAL determined in a sample from said patient at earlier point of time the therapy is being effective.

7. An *in vitro* method for the differential diagnoses of ACLF from acute decompensation of cirrhosis in a subject which comprises:

   a) determining the expression level of NGAL in a sample from said subject; and
   b) comparing the expression level of NGAL obtained in a) with a reference value

   wherein if the expression level of NGAL is increased with respect to the reference value, said subject suffers ACLF.

8. An *in vitro* method for selecting a patient suffering from ACLF for a therapy aimed at treating the effects of liver failure and/or for selecting a patient for liver transplantation which comprises:

   a) determining the expression level of NGAL in a sample from said patient; and
   b) comparing the expression level of NGAL obtained in a) with a reference sample

   wherein if the expression level of NGAL is increased with respect to the reference sample said patient is a candidate for receiving a therapy aimed at treating the effects of liver failure and/or for liver transplantation.

9. The method according to claims 1 to 8, wherein the determination of the expression level of NGAL comprises the determination of the mRNA level of the gene encoding NGAL or a fragment thereof or determining the protein level encoded by said gene or a variant thereof.

10. The method according to claim 9, wherein the determination of protein level is carried out by western blot, ELISA or a protein array.

11. The method according to any of claims 1 to 10, wherein the expression level of NGAL is measured relative to urine creatinine concentration.

12. The method according to any of claims 1 to 11, wherein the sample is a biofluid selected from urine or serum.

13. The method according to any of claims 1 to 12 wherein the patient shows a high MELD score, preferably wherein the MELD score is higher than 20.

14. The method according to any of claims 1 to 13 wherein the patient does not show impairment of kidney function.

15. The use of NGAL for determining the risk of developing ACLF, for determining the prognosis of a subject suffering from ACLF or cirrhosis, for determining the stage or severity of ACLF or cirrhosis in a subject, for monitoring the effect of a therapy in a patient suffering from ACLF, for designing a personalized therapy for a subject suffering from ACLF or for the differential diagnosis of ACLF from acute decompensation of cirrhosis.

Fig. 1

Fig. 2

Europäisches
Patentamt

European
Patent Office

Office européen
des brevets

# EUROPEAN SEARCH REPORT

Application Number

EP 14 38 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GOKHAN GUNGOR ET AL: "Neutrophil gelatinase-associated lipocalin in prediction of mortality in patients with hepatorenal syndrome: a prospective observational study", LIVER INTERNATIONAL, vol. 34, no. 1, 26 January 2014 (2014-01-26), pages 49-57, XP055139097, ISSN: 1478-3223, DOI: 10.1111/liv.12232 * page 51, right-hand column, paragraph 1; figure 2 * | 2-4,9, 12,14,15 | INV. C12Q1/68 G01N33/576 G01N33/68 |
| X | US 2010/233740 A1 (BARASCH JONATHAN [US] ET AL) 16 September 2010 (2010-09-16) * claims 1,5,11-12; figures 1,3 * | 1-3,5-7, 12,15 | |
| X | BARRETO ROGELIO ET AL: "Urinary neutrophil gelatinase-associated lipocalin predicts kidney outcome and death in patients with cirrhosis and bacterial infections", JOURNAL OF HEPATOLOGY, vol. 61, no. 1, 5 March 2014 (2014-03-05), pages 35-42, XP028855573, ISSN: 0168-8278, DOI: 10.1016/J.JHEP.2014.02.023 * abstract * | 2 | |

TECHNICAL FIELDS
SEARCHED        (IPC)

C12Q
G01N

-/--

The present search report has been drawn up for all claims

| Place of search | Date of completion of the search | Examiner |
|---|---|---|
| Munich | 11 September 2014 | Knudsen, Henrik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 14 38 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ELIZABETH C VERNA ET AL: "Urinary Neutrophil Gelatinase-Associated Lipocalin Predicts Mortality and Identifies Acute Kidney Injury in Cirrhosis", DIGESTIVE DISEASES AND SCIENCES, KLUWER ACADEMIC PUBLISHERS-PLENUM PUBLISHERS, NE, vol. 57, no. 9, 6 May 2012 (2012-05-06), pages 2362-2370, XP035104124, ISSN: 1573-2568, DOI: 10.1007/S10620-012-2180-X * figure 2a * | 1,2 | |
| X | A. J. SLACK ET AL: "Predicting the development of acute kidney injury in liver cirrhosis - an analysis of glomerular filtration rate, proteinuria and kidney injury biomarkers", ALIMENTARY PHARMACOLOGY & THERAPEUTICS, vol. 37, no. 10, 12 May 2013 (2013-05-12), pages 989-997, XP055139095, ISSN: 0269-2813, DOI: 10.1111/apt.12299 * page 989 * * page 990, right-hand column * | 1,2 | |
| X | G.A. ROTH ET AL: "Lipocalin-2 Serum Levels Are Increased in Acute Hepatic Failure", TRANSPLANTATION PROCEEDINGS, vol. 45, no. 1, 31 January 2013 (2013-01-31), pages 241-244, XP055139115, ISSN: 0041-1345, DOI: 10.1016/j.transproceed.2012.02.047 * abstract * | 2,9,10,12 | TECHNICAL FIELDS SEARCHED (IPC) |

-/--

The present search report has been drawn up for all claims

| Place of search | Date of completion of the search | Examiner |
|---|---|---|
| Munich | 11 September 2014 | Knudsen, Henrik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

........................................................................................
& : member of the same patent family, corresponding
document

EPO FORM 1503 03.82 (P04C01)

## EP 2 930 247 A1

Europäisches
Patentamt

European
Patent Office

Office européen
des brevets

# EUROPEAN SEARCH REPORT

**Application Number**

EP 14 38 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BARRETO R ET AL: "1019 URINARY NGAL IS USEFUL TO PREDICT CAUSE AND SEVERITY OF KIDNEY FUNCTION IMPAIRMENT AND IS A PROGNOSTIC MARKER IN PATIENTS HOSPITALIZED FOR COMPLICATIONS OF CIRRHOSIS", JOURNAL OF HEPATOLOGY, vol. 58, 1019, April 2013 (2013-04), XP028588156, ISSN: 0168-8278, DOI: 10.1016/S0168-8278(13)61020-X * abstract * | 1,2 | |
| X | ANASS AHMED QASEM ET AL: "Urinary Biomarkers of Acute Kidney Injury in Patients with Liver Cirrhosis", ISRN NEPHROLOGY, vol. 82, no. 5, 6 April 2014 (2014-04-06), pages 945-7, XP055139154, DOI: 10.1378/chest.128.4.2847 * abstract * | 1 | |
| X | PORTAL A J ET AL: "Current measurements of renal function do not reflect true glomerular filtration: a comparison of standard and novel markers of renal function in patients undergoing liver transplant assessment", GUT, vol. 57, no. Suppl. 1, 103, January 2008 (2008-01), pages A39-A40, XP9180065, ANNUAL GENERAL MEETING OF THE BRITISH-SOCIETY-OF-GASTROENTEROLOGY; BRIMINGHAM, UK; MARCH 10 -13, 2008 ISSN: 0017-5749 * abstract * | 8 | **TECHNICAL FIELDS SEARCHED (IPC)** |

-/--

The present search report has been drawn up for all claims

| Place of search | Date of completion of the search | Examiner |
|---|---|---|
| Munich | 11 September 2014 | Knudsen, Henrik |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 14 38 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CLAUDIA FAGUNDES ET AL: "Urinary neutrophil gelatinase-associated lipocalin as biomarker in the differential diagnosis of impairment of kidney function in cirrhosis", JOURNAL OF HEPATOLOGY, ELSEVIER, AMSTERDAM, NL, vol. 57, no. 2, 6 March 2012 (2012-03-06), pages 267-273, XP028426600, ISSN: 0168-8278, DOI: 10.1016/J.JHEP.2012.03.015 [retrieved on 2012-04-17] * abstract * | 1-15 | |
| A | ANDREW J. PORTAL ET AL: "Neutrophil gelatinase-Associated lipocalin predicts acute kidney injury in patients undergoing liver transplantation", LIVER TRANSPLANTATION, vol. 16, no. 11, 28 November 2010 (2010-11-28), pages 1257-1266, XP055139145, ISSN: 1527-6465, DOI: 10.1002/lt.22158 * abstract * | 1-15 | |
| A | TOSHIHISA SEMBA ET AL: "The FLS (Fatty liver Shionogi) mouse reveals local expressions of lipocalin-2, CXCL1 and CXCL9 in the liver with non-alcoholic steatohepatitis", BMC GASTROENTEROLOGY, BIOMED CENTRAL LTD., LONDON, GB, vol. 13, no. 1, 23 July 2013 (2013-07-23), page 120, XP021156272, ISSN: 1471-230X, DOI: 10.1186/1471-230X-13-120 * abstract * | 1-15 | |

TECHNICAL FIELDS
SEARCHED       (IPC)

-/--

The present search report has been drawn up for all claims

| Place of search | Date of completion of the search | Examiner |
|---|---|---|
| Munich | 11 September 2014 | Knudsen, Henrik |

EPO FORM 1503 03.82 (P04C01)

Europäisches
Patentamt

European
Patent Office

Office européen
des brevets

# EUROPEAN SEARCH REPORT

Application Number

EP 14 38 2134

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | X.ARIZA ET AL: "URINARY NEUTROPHIL GELATINASE-ASSOCIATED LIPOCALIN (NGAL) IS A STRONG PREDICTOR OF SHORT-TERM OUTCOME IN PATIENTS WITH ACUTE DECOMPENSATION OF CIRRHOSIS. RELATIONSHIP WITH ACUTE-ON-CHRONIC LIVER FAILURE (ACLF)", JOURNAL OF HEPATOLOGY, vol. 60, O74, 11 April 2014 (2014-04-11), page S30, XP28641750, DOI: 10.1016/S0168-8278(14)60076-3 * abstract * | | |

TECHNICAL FIELDS
SEARCHED      (IPC)

The present search report has been drawn up for all claims

| Place of search | Date of completion of the search | Examiner |
|---|---|---|
| Munich | 11 September 2014 | Knudsen, Henrik |

**ANNEX TO THE EUROPEAN SEARCH REPORT
ON EUROPEAN PATENT APPLICATION NO.**

EP 14 38 2134

This annex lists the patent family members relating to the patent documents cited in the above-mentioned European search report.
The members are as contained in the European Patent Office EDP file on
The European Patent Office is in no way liable for these particulars which are merely given for the purpose of information.

11-09-2014

| Patent document cited in search report | Publication date | Patent family member(s) | Publication date |
|---|---|---|---|
| US 2010233740 A1 | 16-09-2010 | NONE | |

For more details about this annex : see Official Journal of the European Patent Office, No. 12/82

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5804422 A
- US 5374561 A

**Non-patent literature cited in the description**

- **KATOONIZADEH et al.** *Gut,* 2010, vol. 59, 1561-1569
- **CHOLONGITAS et al.** *Nat. Rev. Gastr. And Hepat.,* 2010, vol. 7, 659-668
- **DOWDY ; WEARDEN.** Statistics for Research. John Wiley & Sons, 1983
- **SAMBROOK, J. et al.** Molecular cloning: a Laboratory Manual. Cold Spring Harbor Laboratory Press, vol. 1-3
- **ALTSCHUL, S. et al.** BLASTManual. NCBI NLM NIH
- **ALTSCHUL, S. et al.** *J. Mol. Biol.,* 1990, vol. 215, 403-410
- **PEAKE M et al.** *Clin Biochem Rev,* 2006, vol. 27, 173-184
- *Biol. Chem,* 1936, vol. 113, 515
- **WIESNER R et al.** *Gastroenterology,* 2003, vol. 124, 91-6
- **MOREAU et al.** *Gastroenterology,* 2013, vol. 144, 1426-37
- **KAMATH PS et al.** *Hepatology,* 2007, vol. 445, 797-805
- **DURAND F ; VALLA D.** *Semin Liver,* 2008, vol. 28, 110-22
- **MOREAU R et al.** *Gastroenterology,* 2013, vol. 144, 1426-37
- **ARROYO et al.** *Hepatology,* 1996, vol. 23, 164-76
- **GINÈS P ; SCHRIER RW.** *New England J Med,* 2009, vol. 361, 1279-90
- **KAMATH PS et al.** *Hepatology,* 2007, vol. 45, 797-805
- **DURAND F ; VALLA D.** *Semin Liver Dis,* 2008, vol. 28, 110-22